# FASCICULE DE BREVET EUROPEEN

(11) **EP 1 438 341 B1**
(45) Date de publication et mention de la délivrance du brevet: **08.03.2006**
(21) Numéro de dépôt: 02793224.3
(22) Date de dépôt: 08.11.2002
(51) Int. Cl.: C07K 16/44, C07K 16/06, A61K 39/395, A61P 19/02, A61P 31/00, A61P 37/00

(54) **ANTICORPS STIMULANT LA PRODUCTION D'IL-1RA**
ANTIKÖRPER ZUR STIMULIERUNG DER PRODUKTION VON IL-1RA
ANTIBODY STIMULATING IL-1RA PRODUCTION

(30) Priorité: 09.11.2001 FR 0114518
(43) Date de publication de la demande: 21.07.2004
(73) Titulaire: Laboratoire Français du Fractionnement et des Biotechnologies, 91940 Les Ulis (FR)
(72) Inventeur: BOUREL, Dominique, F-59110 La Madeleine (FR); BRULEY-ROSSET, Martine, F-94210 La Varenne (FR); DHAINAUT, Frédéric, F-91870 Boissy-le-Sec (FR); LIROCHON, Jacky, F-91650 Breuillet (FR)
(74) Mandataire: Warcoin, Jacques
(86) Numéro de dépôt international: PCT/FR2002/003854
(87) Numéro de publication internationale: WO 2003/040188

(56) Documents cités:
- EP-A- 1 059 088
- FR-A- 2 794 460
- TOWBIN H ET AL: "MONOCLONAL ANTIBODY BASED ENZYME-LINKED AND CHEMILUMINESCENT ASSAYS FOR THE HUMAN INTERLEUKIN-1 RECEPTOR ANTAGONIST APPLICATION TO MEASURE HIL-1RA LEVELS IN MONOCYTE CULTURES AND SYNOVIAL FLUIDS" JOURNAL OF IMMUNOLOGICAL METHODS, ELSEVIER SCIENCE PUBLISHERS B.V.,AMSTERDAM, NL, vol. 170, no. 1, 1994, pages 125-135, XP002038694 ISSN: 0022-1759
- AREND W P ET AL: "BIOLOGICAL PROPERTIES OF RECOMBINANT HUMAN MONOCYTE-DERIVED INTERLEUKIN 1 RECEPTOR ANTAGONIST" JOURNAL OF CLINICAL INVESTIGATION, NEW YORK, NY, US, vol. 85, no. 5, 1 mai 1990 (1990-05-01), pages 1694-1697, XP002038689 ISSN: 0021-9738
- DIETRICH G ET AL: "A V REGION-CONNECTED AUTOREATIVE SUBFRACTION OF NORMAL HUMAN SERUM IMMUNOGLOBULIN G*" EUROPEAN JOURNAL OF IMMUNOLOGY, WEINHEIM, DE, vol. 22, no. 7, juillet 1992 (1992-07), pages 1701-1706, XP000877158 ISSN: 0014-2980

## Description

La présente invention se rapporte à l'utilisation d'anticorps naturels de classe IgG capables d'induire une forte production d'IL-1Ra par des monocytes ou des macrophages et à l'utilisation desdits anticorps pour le traitement de maladies inflammatoires ou autoimmunes.

L'IL-1 est une cytokine correspondant à deux protéines (IL-1a et IL-1b) qui jouent un rôle clé dans la réponse inflammatoire (Dinarello CA, Blood, 1996, 87: 2095, 2137). Bien que l'IL-1 soit considérée comme le produit de macrophages activés, elle est également synthétisée par des cellules lymphoïdes et non lymphoïdes comme les cellules vasculaires, épithéliales, cutanées, nerveuses, dendritiques, fibroblastiques et chondrocytaires. Elle est considérée comme un médiateur central de la réponse de l'hôte à l'infection et à d'autres formes de traumatismes.

L'IL-1 est une cytokine multi-fonctionelle agissant sur une grande variété de cellules du système immunitaire mais aussi d'autres systèmes cellulaires. L'IL-1 agit directement sur le système nerveux central, induit l'inhïbition de la sécrétion d'insuline par les cellules des îlots du pancréas et stimule les réponses immunitaires cellulaires, humorales et naturelles.

L'IL-1α et l'IL-1β agissent en se liant à des récepteurs de surface exprimés sur de nombreuses cellules et déclenchent une série de signaux ayant des effets sur la prolifération cellulaire, l'apoptose, l'induction de cytokines, la production de prostaglandines, l'activation de récepteurs et la sécrétion d'enzymes de dégradation. Il existe deux récepteurs qui interagissent avec IL-1a et IL-1b : le récepteur de type 1 (80Kd) exprimé sur les cellules T, les fibroblastes et les cellules épithéliales, qui requiert une interaction avec l'IL1RacP (IL-1 accessory protein) pour transmettre le signal et le récepteur de type II (67Kd) exprimé sur les cellules B, les neutrophiles et les macrophages qui ne peut pas transmettre le signal parce qu'il manque la partie intracellulaire.

Un troisième membre de la famille de l'IL-1, I'IL-1Ra (IL-1 receptor antagonist), agit comme antagoniste naturel de l'IL-la et IL- 1b et interagit avec le récepteur de l'IL-1de type I mais ne transduit pas le signal intracellulaire ou une réponse biologique. Ces trois protéines ont en commun 25 à 30% des acides aminés. L'IL,-1Ra s'associe mal au récepteur de type II.

L'IL-1 est considéré comme un médiateur de la défense de l'hôte contre les infections mais aussi de nombreuses pathologies et un niveau élevé d'IL-1 circulant corrèle avec une variété de situations cliniques et en particulier le choc septique. Son activité est finement régulée au niveau de la transcription, traduction et sécrétion. D'autres mécanismes régulateurs mettent en jeu l'action d'autres cytokines, la différence d'expression des récepteurs à l'EL-1 et la production d'inhibiteur naturel comme l'IL-IRa.

De nombreuses études expérimentales et quelques essais cliniques ont montré le rôle protecteur de l'IL-1Ra dans un certain nombre de pathologies : choc septique, arthrite rhumatoïde, le rejet de greffe, attaque et ischémie cardiaque.
L'IL-1Ra peut agir comme un compétiteur de l'IL-1 et donc représente un agent thérapeutique intéressant. WO 95/16706, WO 99/36541, WO 01/42305, WO 01/41792 et WO 01/42304 décrivent le peptide IL-1Ra, des peptides mutés et leur utilisation pour le traitement de diverses pathologies.

EP 1 059 088 (LFB) décrit une fraction d'IgG ou IgM réagissant avec la myosine, l'actine, la tubuline et le DNP pour le traitement de maladies autoimmunes. Towbin et al, Journal of Imminological Methods, vol 170 n°1, 1994 decrit la sécrétion d'Il-1Ra par les monocytes humains stimulés par des IgG humains décrit la production d'anticorps monoclonaux à partir des lymphocytes transformées par EBV.

Cependant, si les essais thérapeutiques utilisant l'IL-1Ra recombinant soluble ont montré une bonne efficacité de ce produit dans le choc septique, l'arthrite rhumatoïde et la GVH, les doses nécessaires très élevées et la courte demi-vie de l'IL-1Ra recombinant sont des obstacles à son utilisation.

Dès lors, toute stratégie visant à stimuler spécifiquement la production d'IL-1Ra et donc à inhiber l'activité inflammatoire de l'IL-1 présente un intérêt thérapeutique dans le traitement d'un certain nombre de pathologies inflammatoires aiguës ou chroniques comme l'arthrite rhumatoïde , la GVH et autres.

La présente invention porte sur l'utilisation d'anticorps capables de stimuler fortement la production *in vitro* (et *in vivo)* de 1'IL-1Ra (Interleukin 1 receptor antagonist), mais pas celle d'une cytokine pro-inflammatoire 1'IL-1b (Interleukin 1b et 1a). Ces anticorps humains de type IgG ont pour caractéristique de reconnaître en ELISA des protéines du soi telles que l'actine, la myosine, la tubuline, l'ADN ou la protéine basique de la myéline (MBP) mais pas des antigènes vaccinaux comme l'anatoxine tétanique. Ils sont sélectionnés également par leur capacité à se lier à l'haptène Dinitrophenyl ou DNP.

Ces anticorps peuvent donc se substituer avantageusement à l'IL-1Ra pour toutes applications thérapeutiques et répondent aux problèmes évoqués précédemment.

### Description

Ainsi, dans un premier aspect, l'invention se rapporte à des anticorps naturels de classe IgG réagissant avec l'haptène DNP et au moins un autoantigène sélectionné parmi la myosine, l'actine, la protéine basique de la myéline (MBP) et la tubuline, caractérisé en ce qu'il induit une surexpression de l'IL-1Ra au niveau de l'ARNm et de la protéine sécrétée.
Le terme « surexpression » signifie que le taux d'expression de l'IL-1Ra est au moins 50% supérieur au taux d'expression des cellules non stimulées par les anticorps de l'invention.
Par « anticorps naturels de classe IgG», on entend désigner des anticorps, des fractions ou sous-populations d'anticorps ou des anticorps monoclonaux présentant une réactivité vis-à-vis d'antigènes du soi. Les sous-populations d'IgG sont préparées à partir d'Ig polyvalentes ou toute autre fraction intermédiaire obtenue au cours du procédé de fabrication des IgIV à usage thérapeutique.

Avantageusement, de tels anticorps naturels ne réagissent pas avec l'anatoxine tétanique et d'autres antigènes du non soi.

Plus spécifiquement, ils présentent une réactivité vis-à-vis de l'haptène DNP supérieure à 100, 125 ou 150 par rapport aux IgIV, notamment par rapport à TEGELINE®. En outre, ces anticorps présentent un taux de réactivité vis-à-vis d'au moins un autoantigène sélectionné parmi l'ADN, la myosine, la MBP, l'actine et la tubuline supérieur à 50, 75 ou 100 par rapport aux IgIV polyvalentes, notamment par rapport à TEGELINE®. Le taux d'enrichissement peut être calculé par rapport à l'activité des Ig polyvalentes initiales comme valeur contrôle dans le cas de sous-populations d'IgG ou par rapport à un anticorps monoclonal de référence dans le cas où l'anticorps naturel est un anticorps monoclonal.

Dans un deuxième aspect, l'invention porte sur un procédé de préparation d'une sous-population d'IgG définie précédemment caractérisé en ce qu'il comprend les étapes suivantes :
a) préparation d'un support insoluble sur lequel est greffé du DNP-lysine,
b) adsorption d'Ig polyvalentes sur le support obtenu à l'étape a),
c) élution des Ig retenues sur le support de façon à recueillir la fraction interagissant avec le DNP,
d) sélection des fractions présentant une réactivité vis-à-vis d'autoantigènes donnés, et
e) sélection des fractions induisant la surexpression de l'IL-1Ra par les macrophages ou les monocytes.

Le procédé général de préparation d'Ig polyvalentes est connu de l'homme du métier et comporte essentiellement les étapes suivantes :
- Fractionnement du plasma provenant d'un pool de donneurs par précipitation, adsorption et filtration puis ultrafiltration (obtention d'une première fraction "PSO 1"),

Traitement par la pepsine à pH acide, formulation, répartition, et lyophilisation (obtention du produit TEGELINE®),
- Bien entendu, les Ig polyvalentes peuvent être préparées en utilisant d'autres procédés.
Actuellement, la tolérance et l'efficacité des IgG polyvalentes commercialisées, notamment TEGELINE® (LFB, France) sont reconnues en particulier dans le traitement du PTI, de la maladie de Kawasaki et de la rétinochoroïdite de type "Birdshot", pathologies pour lesquelles des AMM ont été obtenues.
On entend par " Ig polyvalentes " dans le cadre de l'invention, des IgG polyvalentes entières, des fragments d'IgG polyvalentes tels que F(ab')2 ou F(ab) et toutes fractions intermédiaires obtenues au cours du procédé de fabrication des IgIV polyvalentes.

L'étape a) peut être mise en oeuvre par greffage de DNP-Lysine sur un support solide, notamment sur un gel de Sepharose®, de Trisacryl®, d'Affiprep® d'Affigel®, gels activés avec CNBr, NHS ou C₅H₈O₂ (glutaraldéhyde). Ainsi, dans le procédé, les IgG déposées sur le support solide obtenu à l'étape a) sont adsorbées soit sous forme d'IgG polyvalentes mises en solution après lyophilisation ou d'IgG polyvalentes sous forme liquide, soit sous forme de fractions intermédiaires obtenues au cours d'un procédé de fabrication d'IgG polyvalentes en tampon phosphate comprenant du NaCl dont la concentration peut varier de 0 M à 3 M.

L'élution des IgG retenues à l'étape b) est effectuée avec un tampon d'ions dissociant la liaison entre les IgG et le DNP, sélectionnés notamment parmi les chaotropes tels que la glycine-HCl ou l'iodure de sodium (NaI), dans des conditions faisant varier le pH et/ou la molarité du tampon.

L'étape d) comprend un test ELISA effectué sur un panel d'autoantigènes sélectionnés notamment parmi l'ADN, l'actine, la myosine, la MPB et la tubuline ou tout autre antigène du soi. Cette étape peut également comprendre une mesure de la réactivité pour un antigène vaccinal, par exemple l'anatoxine tétanique, en prenant le taux d'enrichissement par rapport à l'activité des Ig polyvalentes initiales comme valeur contrôle.

On sélectionne ensuite à l'étape e) des sous-populations possédant les propriétés évoquées ci-dessus, c'est à dire des sous-populations induisant la surexpression de l'ARNm et de la protéine IL-1Ra par les macrophages ou les monocytes. On considère qu'il y a surexpression lors que l' on détecte une augmentation significative de l'expression comparée à l'expression de base observée naturellement dans les macrophages ou les monocytes (par exemple une augmentation supérieure à 50% ou plus (par exemple 100 % ou 200 %).

L'invention porte également sur les sous-populations susceptibles d'être obtenues à partir de ce procédé.

L'invention se rapporte également à un procédé de préparation d'anticorps monoclonaux définis ci-dessus, caractérisé en ce qu'il comprend les étapes suivantes :
a) transformation des lymphocytes B provenant du sang de donneur sain par l'EBV,
b) identification de clones dans chaque puits de culture sécrétant des IgG capables de reconnaître l'haptène DNP et au moins un autoantigène donné par un test ELISA,
c) amplification des clones identifiés à l'étape b),
d) purification des anticorps produits par les clones de l'étape c) et,
e) sélection des anticorps monoclonaux induisant la surexpression de l'IL-1Ra par les macrophages ou les monocytes humains.

Pour améliorer la stabilité de l'expression des Ig naturelles, les clones identifiés à l'étape b) peuvent être fusionnés avec un myélome non sécréteur d'Ig.

Dans ce procédé, l'autoantigène peut être sélectionné parmi l'ADN, l'actine, la myosine, la protéine basique de la myéline (MBP) et la tubuline.

Dans un autre aspect, l'invention vise les clones de cellules B transformées par EBV sélectionnés, fusionnés ou non avec un myélome et amplifiés par le procédé mentionné ci-dessus. Ces clones se caractérisent en ce qu'ils produisent des anticorps monoclonaux naturels et capables d'induire la surexpression de l'IL-1Ra par les macrophages ou les monocytes.

L'invention a également trait aux anticorps monoclonaux obtenus par lesdits clones. De tels anticorps sont capables d'induire la surexpression de l'IL-1Ra par les macrophages ou les monocytes.

Dans un mode supplémentaire de réalisation, l'invention porte sur une composition comprenant un anticorps naturel défini ci-dessus (sous-population d'anticorps ou anticorps monoclonal) et un véhicule pharmaceutiquement acceptable. Avantageusement, cette composition est adaptée à une administration par voie intraveineuse, subcutanée ou intramusculaire.

L'invention vise également l'utilisation d'un anticorps naturel mentionné ci-dessus pour la préparation d'un médicament. De préférence, ce médicament est destiné au traitement et à la prévention des maladies inflammatoires. Il peut être utile pour le traitement et la prévention de la septicémie et des chocs septiques, pour le traitement et la prévention de l'arthrite, de l'arthrite rhumatoïde, de la polyarthrite, de l'ostéoporose, des maladies inflammatoires de l'intestin, des maladies autoimmunes, des rejets de greffes, des ischémies, des traumatismes, notamment des traumatismes du cerveau, du psoriasis, des dermatoses, des resténoses, des maladies respiratoires, des rhinites allergiques, des allergies et du cancer.

### Légendes

**Figure 1 :** Capacité inhibitrice de TEGELINE® et de la fraction anti-DNP sur la prolifération de lymphocytes humains stimulés en culture lymphocytaire mixte (CLM).
**Figure 2 A-B-C** : Effet de TEGELINE® et de la fraction anti-DNP sur la production de cytokines par les PBL humains stimulés en CML.
**Figure 3** : Sécrétion d'IL1Ra par des macrophages de souris in vitro.
**Figure 4 :** Production d'IL-1Ra par les cellules THP1 stimulées par différentes doses de TEGELINE® ou de la fraction anti-DNP.
**Figure 5 :** Capacité inhibitrice de TEGELINE® et d'anticorps monoclonaux anti-D sur la prolifération de lymphocytes humains stimulés en CML.
**Figure 6 A-B :** Effet de TEGELINE® et d'anticorps monoclonaux anti-D sur la production de cytokines par des PBL humains stimulés en CML.
**Figure 7** : Production d'IL-1ra par les cellules THPA stimulées par différentes doses (25, 50, 100 µg/ml) d'anticorps monoclonaux anti-D

| Anticorps monoclonaux | Anti-DNP |
|---|---|
| D41/96 | (+++) |
| D32/97 | (+) |
| D35/97 | (-) |
| D83/97 | (-) |

**Figure 8** : Dosage de 1'IL-1ra en ELISA dans le surnageant de culture de cellules THP1 stimulées par 25 µg/ml de TEGELINE® ou par IgG provenant de cultures de cellules B EBV+ C1 (DNP++) ou C2 (DNP-)

### Exemple 1 : Obtention d'une fraction d'IgG anti-DNP à partir d'immunoglobulines polyclonales issues d'un pool de plasma provenant de donneurs sains, (IVIg).

Il a été montré précédemment (FR 99/16632) que le passage sur un immunoadsorbant NHS-Affiprep couplé au DNP-Lysine d'une préparation d'IVIg (TEGELINE®, LFB) permet d'obtenir par élution avec de l'iodure de sodium (NaI), un éluat représentant 0.13% des IgG déposées. Cet éluat possède une forte réactivité en ELISA pour le DNP-albumine et pour de nombreux auto antigènes tels que l'actine, la myosine, le MBP, l'ADN et la tubuline mais pas l'anatoxine tétanique (tableau 1).

**Tableau 1 : Réactivité en ELISA de la fraction anti-DNP par rapport à TEGELINE® vis-à-vis de plusieurs antigènes**

| **Taux d'enrichissement de la fraction anti-DNP par rapport à TEGELINE®** | | | | | | | |
|---|---|---|---|---|---|---|---|
| Ana. Téta. | F(ab')2 | DNP | Actine | Myosine | MBP | ADN | Tubuline |
| 4,8 ± 2,2 | 4,5 ±1,3 | 235 ±100 | 158 ±64 | 110 ± 33 | 94 ± 66 | 26.0±17 | 238 ± 108 |

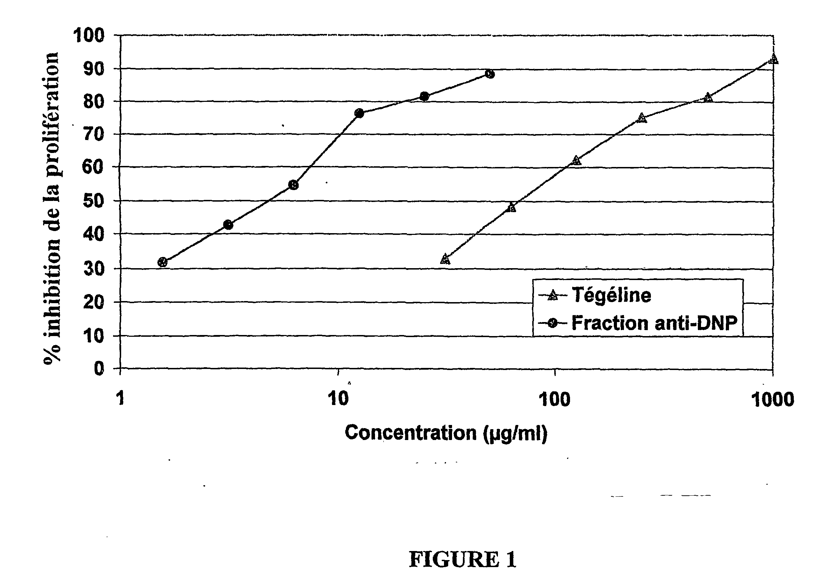

Cette fraction anti-DNP est capable d'inhiber dix à vingt fois plus que les IgG de départ (TEGELINE®) la prolifération de deux suspensions de PBL humains incompatibles en CML (figure 1).

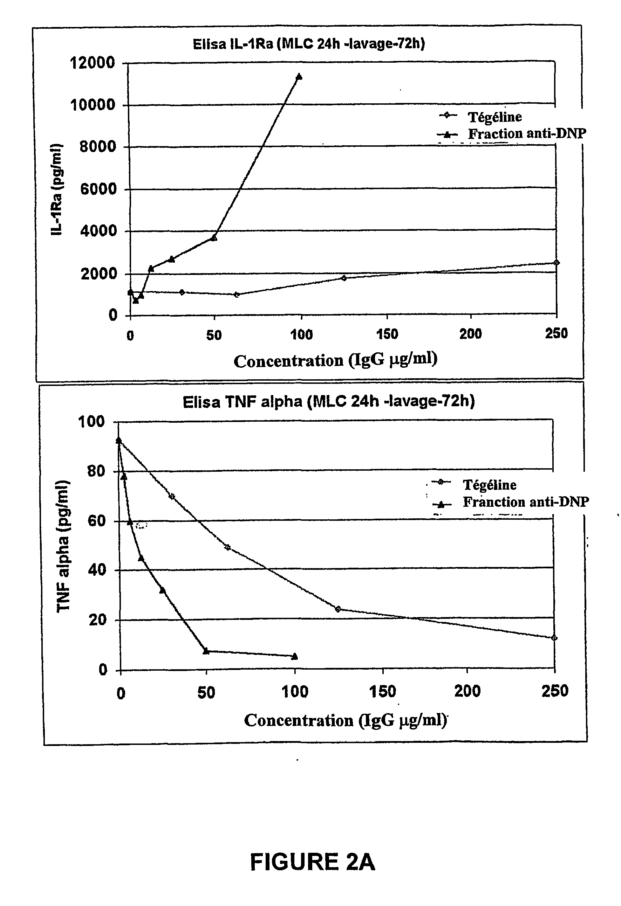

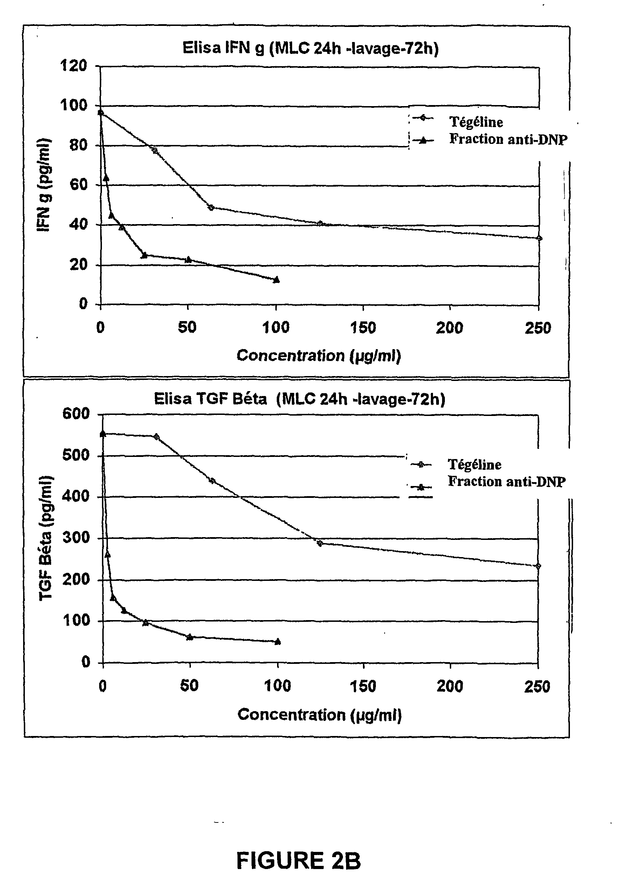

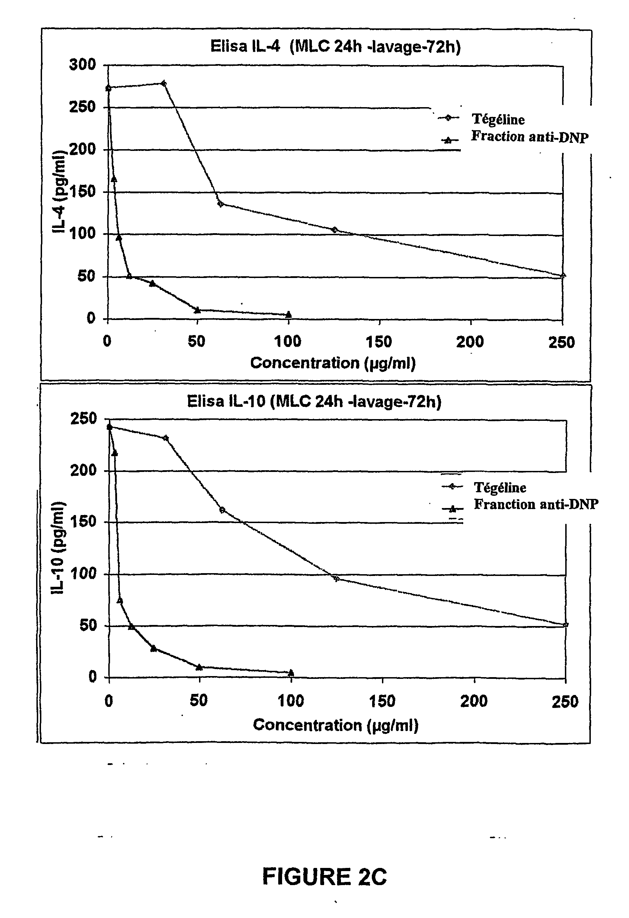

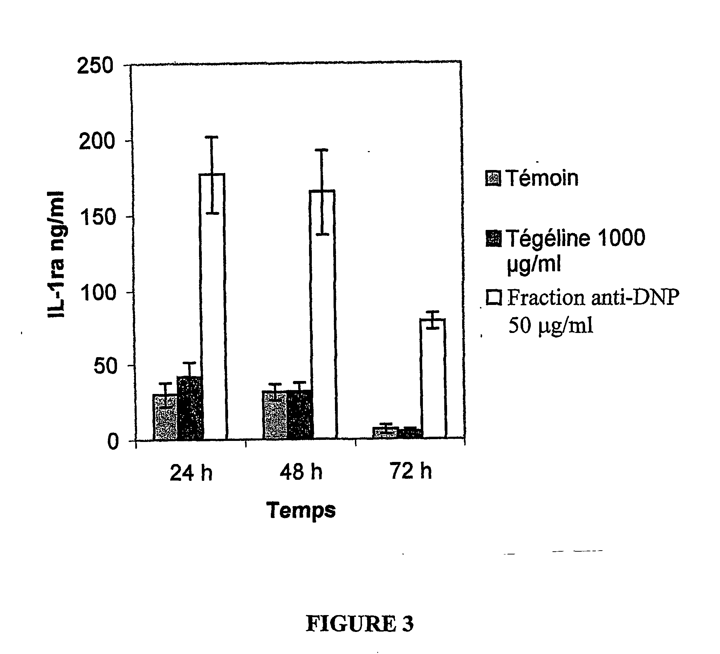

L'analyse en ELISA de la présence de plusieurs cytokines dans le surnageant des CML montrent que la sécrétion d'IFNg, d'IL-4, d'IL-10, de TNFa et de TGFb est plus fortement inhibée par la fraction anti-DNP que par TEGELINE®. En revanche, la fraction anti-DNP stimule très fortement la synthèse d'IL-1Ra (figures 2).
L'incubation de 2 x 10⁶ de macrophages péritonéaux de souris *in vitro* avec la fraction anti-DNP pendant 24h, 48h et 72h provoque la libération d'IL-IRa de souris ; par contre, l'incubation avec TEGELINE® n'a aucun effet (figure 3).

La mesure de la quantité des ARNm de l'IL-1Ra dans les cellules THP1 (lignées de cellules monocytaires humaines) a été réalisée par PCR quantitative. Le niveau d'expression de l'ARNm de l'IL-1Ra par rapport au niveau d'expression de l'ARNm de l'actine est très augmenté après incubation des cellules THP1 en présence de la fraction anti DNP mais pas de TEGELINE® (tableau 2). L'expression de l'ARNm de TNFα est également augmentée en présence de la fraction mais dans une moins moindre mesure.

**Tableau 2 : Effet de TEGELINE® et de la fraction anti-DNP sur la synthèse de l'ARNm du TNFα et de l'IL-1Ra par la cellule THP1.**

| **Echantillons (dosage par RT-PCR quantitative)** | **ARNm TNFα / ARNm β actine** | **ARNm IL-1RA /ARNm β actine** |
|---|---|---|
| Cellules THP 1 | 4 | 2 |
| Cellules THP1 (+ TEGELINE® 50 µg/ml) | 1 | 9 |
| Cellules THP1 (+ fraction anti-DNP 50 µg/ml) | 21 | 148 |

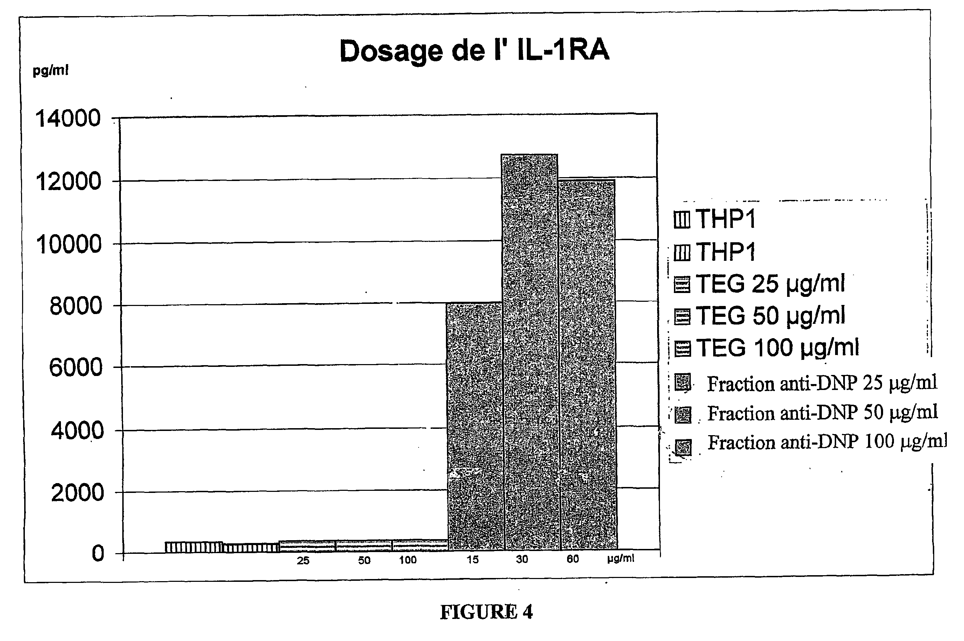

De même, le surnageant des cellules THP1 stimulées par la fraction anti-DNP contient d'importante quantité d'IL1-Ra alors qu'aux mêmes concentrations, TEGELINE® n'induit aucune production de cette cytokine (figure 4).

Des expériences précédentes (brevet n° 99 16632) avaient démontré que cette fraction anti-DNP administrée in vivo protégeait contre le développement de la polyarthrite induite par le collagène, de l'encéphalomyélite expérimentale auto immune (EAE) induite par la MBP chez le rat et du diabète chez la souris NOD.
Cette activité protectrice est supérieure après injection des IgG anti-DNP par rapport aux IgIV non-fractionnées. Ces anticorps naturels pourraient représenter un traitement efficace de certaines pathologies inflammatoires.

### Exemple 2: Obtention d'anticorps humains monoclonaux naturels ayant la propriété de stimuler la production d'IL1-Ra.

### Exemple 2.1: Production d'anticorps monoclonaux à partir de lymphocytes du sang périphérique humain provenant d'un donneur immunisé.

Une série d'anticorps monoclonaux humains (Acm) ont été obtenus après transformation par le virus EBV de lymphocytes B provenant du sang de donneurs immunisés par des globules rouges Rh+. Ces anticorps sont tous de type IgG 1 et ont la propriété d'agglutiner les globules rouges Rh+. Ils sont donc spécifiques de l'antigène D.

Quatorze Acm anti-D ont été testés pour leur capacité à reconnaître en ELISA l'haptène DNP et les autoantigènes décrits ci-dessus.
Sur les quatorze Acm, seuls deux Acm D41 et D32 reconnaissent le DNP mais seul D41 se lie aux autoantigènes mais pas à l'anatoxine tétanique. Les autres Acm ne reconnaissent aucun des antigènes de manière supérieure à TEGELINE (tableau 3).

**Tableau 3 : Réactivité en ELISA des anticorps monoclonaux anti-D par rapport à TEGELINE® vis-à-vis de plusieurs antigènes.**

| **Echantillons** | **Taux d'enrichissement de l'activité par rapport à TEGELINE®** | | | | | | | |
|---|---|---|---|---|---|---|---|---|
| | Anatoxine | DNP-Alb | DNA | F(ab)' 2 | Actine | Myosine | MBP | Tubuline |
| D16/96 P1 | < 0,1 | 2,7 | 4 | <0,1 | 1,86 | <0,1 | <0,1 | < 0,1 |
| D39/96 P4 | < 0,1 | 3,52 | 0 | <0,1 | 1,2 | <0,1 | < 0,1 | <0,1 |
| D12/96 P5 | <0,1 | 2,28 | 0 | <0,1 | 3,1 | <0,1 | < 0,1 | 1,69 |
| D12/96 P7 | <0,1 | 1,06 | 0 | <0,1 | 1,75 | <0,1 | <0,1 | <0,1 |
| D41/96P6/1 | <0,1 | **213** | **400** | <0,1 | **119** | <0,1 | <0,1 | **133** |
| D32/97 P1 | < 0,1 | **154** | 3,4 | <0,1 | 2,36 | < 0,1 | <0,1 | <0,1 . |
| D49/97 P3 | <0,1 | 0,38 | 0,04 | <0,1 | 0,43 | <0,1 | < 0,1 | <0,1 |
| D35/97 P3 | <0,1 | 0,03 | 0 | <0,1 | 0,08 | <0,1 | <0,1 | <0,1 |
| D83/97 P4 | < 0,1 | 0,29 | 0 | < 0,1 | 0,09 | < 0,1 | 0,06 | < 0,1 |
| Df5/97 P6 | <0,1 | 0,19 | 0 | <0,1 | 0,17 | < 0,1 | 0,36 | <0,1 |
| D83/97 P7 | <0,1 | 3,45 | 0 | <0,1 | 0,24 | <0,1 | 0,51 | <0,1 |
| D31/97 P8 | <0,1 | 4,15 | 2,75 | <0,1 | 3 | <0,1 | 0,71 | 2,18 |
| Pf210 | 0.16 | 8,8 | 8,4 | NT | 2,8 | 2,5 | 0,15 | 6,1 |

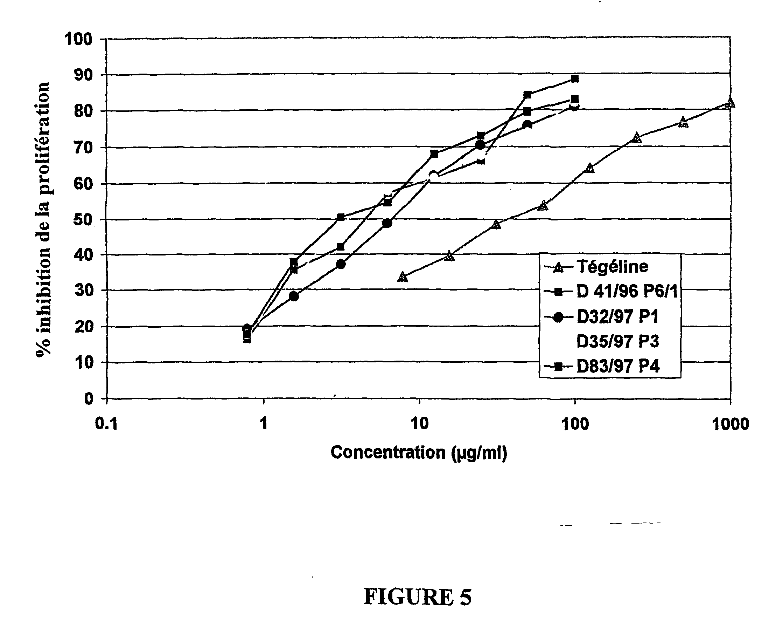

La capacité des Acm anti-D à inhiber la prolifération de lymphocytes humains incompatibles en CML est supérieure à celle de TEGELINE® pour tous les anticorps qu'ils réagissent avec le DNP ou non (figure 5).

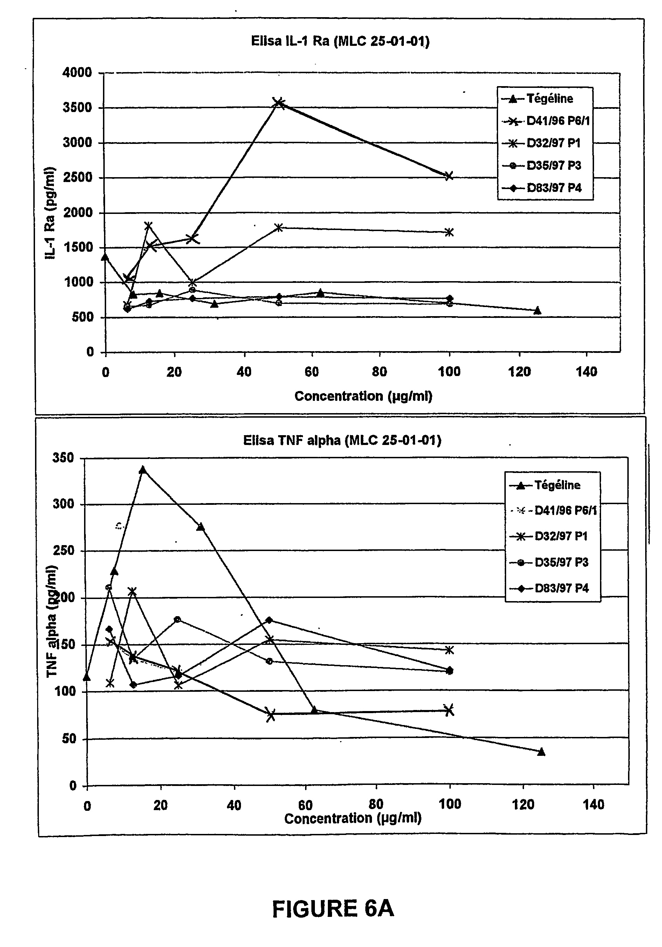

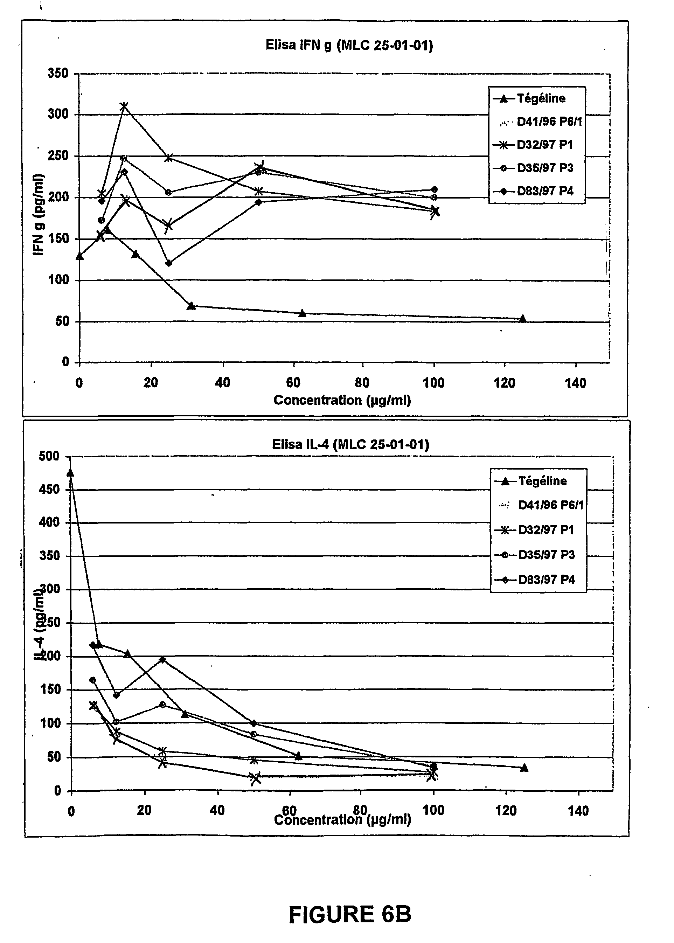

L'étude de la libération de cytokines dans le surnageant des CML montrent que, seuls les deux Acm D41 et D32 réagissant avec le DNP, provoquent une synthèse accrue d'IL-1Ra proportionnellement à leur réactivité au DNP (figure 6). La synthèse d'autres cytokines est soit diminuée (IL-4) soit non modifiée (INFg et TNFa) par les Acm quelle que soit leur réactivité (figures 6).

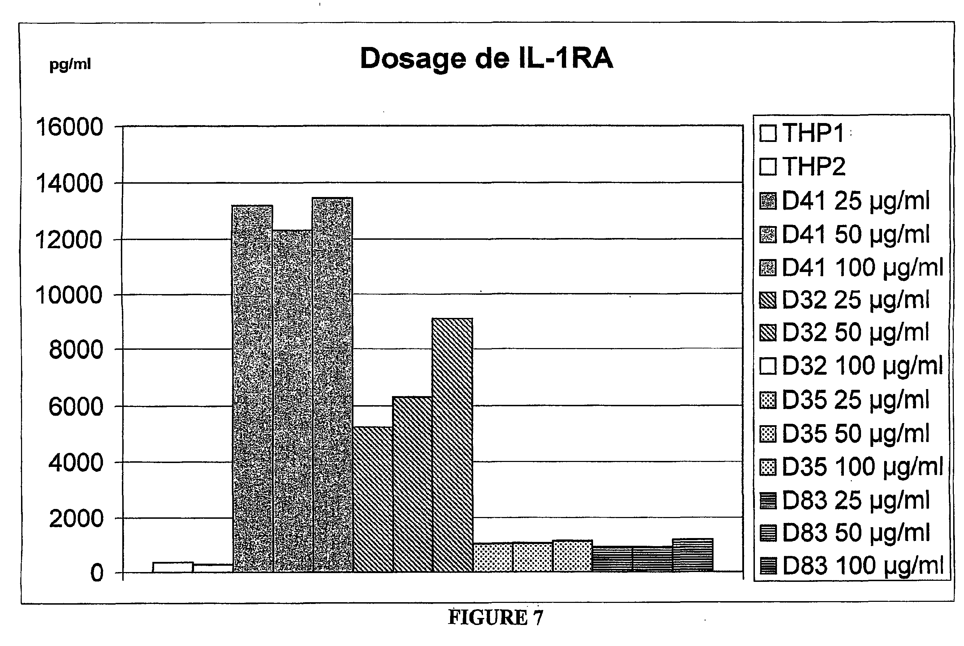

Le surnageant des cellules THP1 stimulées par l'Acm D41 et D32 contient d'importante quantité d'IL1-Ra alors qu'aux mêmes concentrations, l'Acm D35 et D83 n'induisent qu'une faible production de cette cytokine (figure 7).

### Exemple 2.2 : Production d'anticorps monoclonaux à partir de lymphocytes du sang périphérique humain provenant d'un donneur sain.

Des lymphocytes B provenant du sang de donneurs sains ont été transformés par l'EBV et mis en culture à raison de 10⁵ cellules par puits. Les surnageants des puits ont été testés pour la présence d'IgG capables de reconnaître en ELISA l'haptène DNP et l'actine. Les quatre puits les plus positifs ont été poolés (C1) et clonés par dilution à 1000, 100, 50 et 10 cellules par puits. La même approche a été utilisée pour sélectionner des puits négatifs en activité anti-DNP et actine (C2) (Tableau 4).

**Tableau 4 : Réactivité en ELISA d'IgG purifiées provenant de surnageants de culture de cellules humaines B EBV+ vis-à-vis de plusieurs antigènes.**

| **Echantillons** | | **Taux d'enrichissement de l'activité par rapport à TEGELINE®** | | | | | | |
|---|---|---|---|---|---|---|---|---|
| | | Anatoxine | DNP-Alb | F(ab)'2 | Actine | Myosine | MBP | Tubuline |
| | Préparation1 | 1,2 | 40 | 6,1 | 16 | 126 | 27 | 56 |
| C1 | | | | | | | | |
| | Préparation2 | 5,8 | 115 | 19 | 96 | 392 | 276 | 212 |
| | Préparation | 0 | 0,9 | 0 | 1,1 | 0 | 0 | 0 |
| C2 | | | | | | | | |
| | Préparation2 | 0,2 | 3,9 | 0,1 | 6,2 | 5 | 1,4 | 5,1 |

Les cellules des puits C1 et C2 ont été amplifiées pour obtenir des quantités d'anticorps suffisantes pour être purifiés sur protéine G. Les anticorps obtenus sont de type IgG et le tableau 4 présentent les résultats de deux purifications réalisées à partir de surnageants prélevés à J+7 (préparation 1) et J+14 (préparation 2) et montrent que les IgG purifiées du puits C1 reconnaissent le DNP et sont réactives pour un certain nombre d'autoantigènes. Par contre, les IgG du puits C2 ne possèdent aucune activité enrichie par rapport à TEGELINE®.

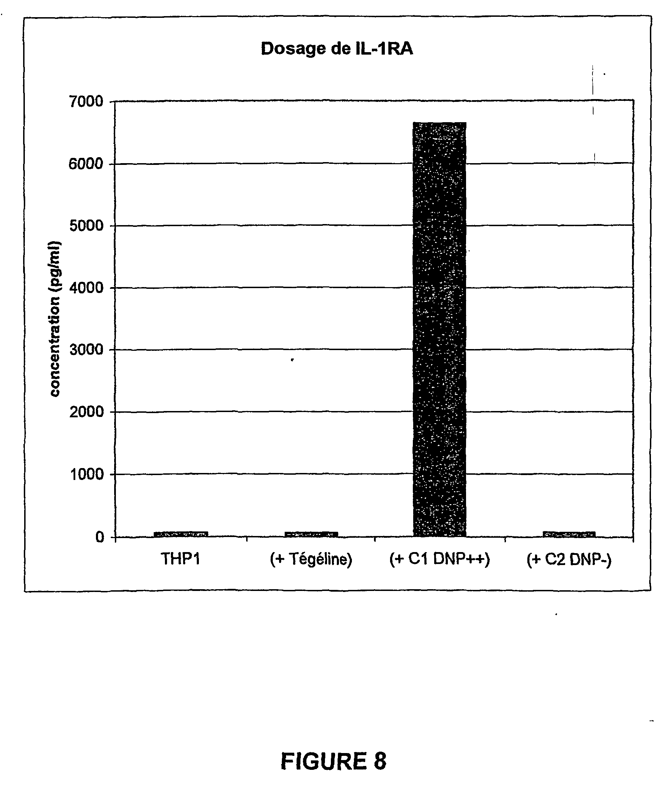

Les IgG purifiées à partir des deux puits C1 et C2 ont été incubées à la dose de 25 µg/ml en présence de cellules THP1. La croissance cellulaire est inhibée en présence des IgG de C1 mais pas de C2 (données non présentées) et seules les IgG de C1 stimulent la production in vitro d'IL-1Ra par les cellules THP1 (figure 8).

Les cellules des puits C1 et C2 sont en cours de clonage à 0.5 cellules par puits pour obtenir des IgG monoclonales.

Tous ces résultats montrent une corrélation positive entre la réactivité d'anticorps de type IgG vis-à-vis de plusieurs auto antigènes et la capacité accrue d'induire la synthèse d'IL-1Ra qu'ils soient polyclonaux ou monoclonaux par des monocytes ou des macrophages.

## Revendications

1. Anticorps monoclonal de classe IgG réagissant avec l'haptène DNP et au moins un autoantigène sélectionné parmi la myosine, l'actine, la protéine basique de la myéline (MBP) et la tubuline, **caractérisé en ce qu'**il induit une surexpression de l'IL-1Ra dans les macrophages ou les monocytes.

2. Anticorps selon la revendication 1, **caractérisé en ce qu'**il ne réagit pas avec l'anatoxine tétanique.

3. Anticorps selon l'une des revendications 1 et 2 ayant un taux de réactivité vis-à-vis de l'haptène DNP supérieur à 100 par rapport aux IgIV, notamment par rapport à TEGELINE®.

4. Anticorps selon l'une des revendications 1 à 3 ayant un taux de réactivité vis-à-vis d'au moins un autoantigène sélectionné parmi la myosine, l'actine, le MBP et la tubuline supérieur à 50 par rapport aux IgIV polyvalentes, notamment par rapport à TEGELINE®.

5. Procédé de préparation d'anticorps monoclonaux définis aux revendications 1 à 4, **caractérisé en ce qu'**il comprend les étapes suivantes :
a) transformation des lymphocytes B provenant du sang de donneur sain par l'EBV,
b) identification de clones dans chaque puits de culture sécrétant des IgG capables de reconnaître l'haptène DNP et au moins un autoantigène donné par un test ELISA,
c) amplification des clones identifiés à l'étape b),
d) purification des anticorps produits par les clones de l'étape c) et,
sélection des anticorps monoclonaux induisant la surexpression de l'IL-1Ra par les macrophages ou les monocytes humains.

6. Procédé selon la revendication 5 **caractérisé en ce que** l'autoantigène est sélectionné parmi l'ADN, l'actine, la myosine, la protéine basique de la myéline (MBP) et la tubuline.

7. Procédé selon l'une des revendications 5 et 6 **caractérisé en ce que** les clones identifiés à l'étape b) sont fusionnés avec un myélome non sécréteur d'Ig.

8. Anticorps monoclonal susceptible d'être obtenus à partir du procédé selon l'une des revendications 5 à 7 **caractérisé en ce qu'**il est capable d'induire la surexpression de l'IL-1Ra par les macrophages ou les monocytes.

9. Clones de lymphocytes B EBV identifiés et amplifiés par le procédé selon l'une des revendications 5 à 7 **caractérisés en ce qu'**ils produisent des anticorps monoclonaux capables d'induire la surexpression de l'IL-1Ra par les macrophages ou les monocytes.

10. Anticorps monoclonal obtenu par un clone selon la revendication 9 **caractérisé en ce qu'**il est capable d'induire la surexpression de l'IL-1Ra par les macrophages ou les monocytes.

11. Composition comprenant un anticorps selon l'une des revendications 1 à 4, 8 et 10 et un véhicule pharmaceutiquement acceptable.

12. Composition selon la revendication 11, **caractérisée en ce qu'**elle est adaptée à une administration par voie intraveineuse, subcutanée ou intramusculaire.

13. Procédé de préparation d'une sous-population d'IgG **caractérisé en ce qu'**il comprend les étapes suivantes :
a) préparation d'un support insoluble sur lequel est greffé du DNP-lysine,
b) adsorption d'Ig polyvalentes sur le support obtenu à l'étape a),
c) élution des Ig retenues sur la partie DNP liée au support de façon à recueillir la fraction interagissant avec le DNP,
d) sélection des fractions présentant une réactivité vis-à-vis d'autoantigènes donnés, et
e) sélection des fractions induisant la surexpression de l'IL-1Ra par les macrophages ou les monocytes au niveau de l'ARNm et de la protéine sécrétée.

14. Procédé selon la revendication 13 **caractérisé en ce que** l'étape d) comprend une mesure de la réactivité pour l'anatoxine tétanique en prenant le taux d'enrichissement par rapport à l'activité des Ig polyvalentes initiales comme valeur contrôle.

15. Procédé selon l'une des revendications 13 et 14 **caractérisé en ce que** l'étape d) comprend un test ELISA effectué sur un panel d'autoantigènes sélectionnés notamment parmi l'ADN, l'actine, la myosine, la protéine basique de la myéline (MBP) et la tubuline.

16. Procédé selon l'une des revendications 13 à 15 **caractérisé en ce que** les Ig déposées sur le support solide obtenu à l'étape a) sont adsorbées soit sous forme d'IgG polyvalentes lyophilisées et remises en solution ou sous forme liquide, soit sous forme de fractions intermédiaires obtenues au cours d'un procédé de fabrication d'IgG polyvalentes en tampon phosphate comprenant du NaCl dont la concentration peut varier de 0 M à 3 M.

17. Utilisation d'une sous-population d'IgG susceptible d'être obtenu par le procédé selon l'une des revendication 13 à 16 pour la fabrication d'un médicament pour induire une surexpression de l'IL-1Ra dans les macrophages ou les monocytes.

18. Utilisation selon la revendication 17 **caractérisée en ce que** la sous-population d'IgG est préparée à partir d'Ig polyvalentes ou toute autre fraction intermédiaire obtenue au cours du procédé de fabrication des IgIV à usage thérapeutique.

19. Utilisation d'un anticorps monoclonal selon l'une des revendications 1 à 4, 8 et 10, pour la préparation d'un médicament.

20. Utilisation selon la revendication 19 pour la préparation d'un médicament destiné au traitement et la prévention des maladies inflammatoires.

21. Utilisation selon la revendication 19 pour la préparation d'un médicament destiné au traitement et la prévention de la septicémie et des chocs septiques.

22. Utilisation selon la revendication 19 pour la préparation d'un médicament destiné au traitement et la prévention de l'arthrite, de l'arthrite rhumatoïde, de la polyarthrite, de l'ostéoporose, des maladies inflammatoires de l'intestin, des maladies autoimmunes, des rejets de greffes, des ischémies, des traumatismes, notamment des traumatismes du cerveau, du psoriasis, des dermatoses, des resténoses, des maladies respiratoires, des rhinites allergiques, des allergies et du cancer.

23. Utilisation selon la revendication 17 ou 18 pour la préparation d'un médicament destiné au traitement et la prévention des maladies inflammatoires, de la septicémie et des chocs septiques, de l'arthrite, de l'arthrite rhumatoïde, de la polyarthrite, de l'ostéoporose, des maladies inflammatoires de l'intestin, des ischémies, des traumatismes, notamment des traumatismes du cerveau, du psoriasis, des dermatoses, des resténoses, des maladies respiratoires, des rhinites allergiques, des allergies et du cancer.

## Patentansprüche

1. Monoklonaler Antikörper der lgG-Klasse, der mit dem Hapten DNP und mindestens einem Autoantigen reagiert, das ausgewählt ist aus Myosin, Actin, basischem Myelin-Protein (MBP) und Tubulin, **dadurch gekennzeichnet, dass** er eine Überexpression von IL-1Ra in Makrophagen oder Monozyten induziert.

2. Antikörper nach Anspruch 1, **dadurch gekennzeichnet, dass** er nicht mit Tetanus-Anatoxin reagiert.

3. Antikörper nach einem der Ansprüche 1 und 2 mit einem Reaktivitätsverhältnis gegenüber dem Hapten DNP von mehr als 100, bezogen auf die IVIg, insbesondere bezogen auf TEGELINE®.

4. Antikörper nach einem der Ansprüche 1 bis 3 mit einem Reaktivitätsprozentsatz gegenüber mindestens einem Autoantigen, das aus Myosin, Actin, MBP und Tubulin ausgewählt ist, von mehr als 50, bezogen auf polyvalente IVIg, insbesondere bezogen auf TEGELINE®.

5. Verfahren zur Herstellung von in den Ansprüchen 1 bis 4 definierten monoklonalen Antikörpern, **dadurch gekennzeichnet, dass** es die folgenden Schritte umfasst:
a) Transformation von B-Lymphozyten, die aus dem Blut eines gesunden Donors abstammen, durch das EBV,
b) Identifikation von Klonen in jeder Kulturvertiefung, die IgG sekretieren, welche das Hapten DNP und mindestens ein gegebenes Autoantigen erkennen können, mittels eines ELISA-Tests,
c) Amplifikation der im Schritt b) identifizierten Klone,
d) Reinigung der Antikörper, die durch die Klone des Schrittes c) produziert werden, und
Selektion der monoklonalen Antikörper, welche die Überexpression von IL-1Ra durch menschliche Makrophagen oder Monozyten induzieren.

6. Verfahren nach Anspruch 5, **dadurch gekennzeichnet, dass** das Autoantigen aus DNA, Actin, Myosin, basischem Myelin-Protein (MBP) und Tubulin ausgewählt ist.

7. Verfahren nach einem der Ansprüche 5 und 6, **dadurch gekennzeichnet, dass** die im Schritt b) identifizierten Klone mit einem Myelom fusioniert sind, das kein lg sekretiert.

8. Monoklonaler Antikörper, erhältlich aus dem Verfahren nach einem der Ansprüche 5 bis 7, **dadurch gekennzeichnet, dass** er die Überexpression von IL-1Ra durch Makrophagen oder Monozyten induzieren kann.

9. Klone von EBV-B-Lymphozyten, identifiziert und amplifiziert durch das Verfahren nach einem der Ansprüche 5 bis 7, **dadurch gekennzeichnet, dass** sie monoklonale Antikörper produzieren, welche die Überexpression von IL-1Ra durch Makrophagen oder Monozyten induzieren können.

10. Monoklonaler Antikörper, erhalten durch einen Klon nach Anspruch 9, **dadurch gekennzeichnet, dass** er die Überexpression von IL-1Ra durch Makrophagen oder Monozyten induzieren kann.

11. Zusammensetzung, umfassend einen Antikörper nach einem der Ansprüche 1 bis 4, 8 und 10 und ein pharmazeutisch annehmbares Vehikel.

12. Zusammensetzung nach Anspruch 11, **dadurch gekennzeichnet, dass** sie an eine Verabreichung auf intravenösem, subkutanem oder intramuskulärem Weg angepasst ist.

13. Verfahren zur Herstellung einer lgG-Unterpopulation, **dadurch gekennzeichnet, dass** es die folgenden Schritte umfasst:
a) Herstellung eines unlöslichen Trägers, auf den DNP-Lysin gepfropft ist,
b) Adsorption von polyvalenten lg auf den im Schritt a) erhaltenen Träger,
c) Elution der lg, die auf dem am Träger gebundenen DNP-Teil zurückgehalten sind, auf solche Weise, dass die mit DNP wechselwirkende Fraktion erhalten wird,
d) Auswahl von Fraktionen, die eine Reaktivität gegenüber gegebenen Autoantigenen aufweisen, und
e) Auswahl von Fraktionen, welche die Überexpression von IL-1Ra durch Makrophagen oder Monozyten induzieren, auf der Grundlage der mRNA und des sekretierten Proteins.

14. Verfahren nach Anspruch 13, **dadurch gekennzeichnet, dass** der Schritt d) eine Messung der Reaktivität gegenüber Tetanus-Anatoxin umfasst, indem das Anreicherungsverhältnis, bezogen auf die Aktivität der anfänglichen polyvalenten lg, als Kontrollwert genommen wird.

15. Verfahren nach einem der Ansprüche 13 und 14, **dadurch gekennzeichnet, dass** der Schritt d) einen ELISA-Test umfasst, der bei einer Gruppe von Autoantigenen bewirkt wird, die insbesondere aus DNA, Actin, Myosin, basischem Myelin-Protein (MBP) und Tubulin ausgewählt sind.

16. Verfahren nach einem der Ansprüche 13 bis 15, **dadurch gekennzeichnet, dass** die lg, die auf dem im Schritt a) erhaltenen festen Träger abgeschieden sind, entweder in Form von polyvalenten lyophilisierten und wieder gelösten IgG oder in flüssiger Form oder in Form von Zwischenfraktionen adsorbiert werden, welche im Lauf eines Herstellungsverfahrens von polyvalenten IgG in Phosphat-Puffer, der NaCl umfasst, dessen Konzentration von 0 M bis 3 M variieren kann, erhalten werden.

17. Verwendung einer Unterpopulation von IgG, erhältlich durch das Verfahren nach einem der Ansprüche 13 bis 16, für die Herstellung eines Medikaments, um eine Überexpression von IL-1Ra in Makrophagen oder Monozyten zu induzieren.

18. Verwendung nach Anspruch 17, **dadurch gekennzeichnet, dass** die Unterpopulation von IgG aus polyvalenten Ig oder jeder anderen Zwischenfraktion hergestellt wird, welche im Lauf des Verfahrens zur Herstellung von IVIg zur therapeutischen Verwendung erhalten wird.

19. Verwendung eines monoklonalen Antikörpers nach einem der Ansprüche 1 bis 4, 8 und 10 für die Herstellung eines Medikaments.

20. Verwendung nach Anspruch 19 für die Herstellung eines Medikaments, das zur Behandlung und Verhütung von entzündlichen Krankheiten bestimmt ist.

21. Verwendung nach Anspruch 19 für die Herstellung eines Medikaments, das zur Behandlung und Verhütung von Septikämie und septischen Schocks bestimmt ist.

22. Verwendung nach Anspruch 19 für die Herstellung eines Medikaments, das zur Behandlung und Verhütung von Arthritis, rheumatoider Arthritis, Polyarthritis, Osteoporose, entzündlichen Darmerkrankungen, AutoimmunKrankheiten, Transplantat-Abstoßungen, lschämien, Traumen, insbesondere Gehirntraumen, Psoriasis, Dermatosen, Restenosen, Atemwegserkrankungen, allergischer Rhinitiden, Allergien und Krebs bestimmt ist.

23. Verwendung nach Anspruch 17 oder 18 für die Herstellung eines Medikaments, das zur Behandlung und Verhütung von entzündlichen Erkrankungen, Septikämie und septischen Schocks, Arthritis, rheumatoider Arthritis, Polyarthritis, Osteoporose, entzündlichen Darmerkrankungen, lschämien, Traumen, insbesondere Gehirntraumen, Psoriasis, Dermatosen, Restenosen, Atemwegserkrankungen, allergischer Rhinitiden, Allergien und Krebs bestimmt ist.

## Claims

1. Monoclonal IgG class antibody which reacts with the hapten DNP and at least one autoantigen selected from myosin, actin, myelin basic protein (MBP) and tubulin, **characterized in that** it induces overexpression of IL-1Ra in macrophages or monocytes.

2. Antibody according to Claim 1, **characterized in that** it does not react with tetanus toxoid.

3. Antibody according to either of Claims 1 and 2, having a degree of reactivity with respect to the hapten DNP which is greater than 100 relative to the IVIgs, in particular to Tegeline®.

4. Antibody according to one of Claims 1 to 3, having a degree of reactivity with respect to at least one autoantigen selected from myosin, actin, MBP and tubulin, which is greater than 50 relative to the polyvalent IVIgs, in particular relative to Tegeline®.

5. Method for preparing monoclonal antibodies defined in Claims 1 to 4, **characterized in that** it comprises the following steps:
a) transforming B lymphocytes originating from the blood of a normal donor, with EBV,
b) identifying clones, in each culture well, secreting IgGs capable of recognizing the hapten DNP and at least one given autoantigen, using an ELISA assay,
c) amplifying the clones identified in step b),
d) purifying the antibodies produced by the clones of step c), and
selecting the monoclonal antibodies which induce overexpression of IL-1Ra by human macrophages or monocytes.

6. Method according to Claim 5, **characterized in that** the autoantigen is selected from DNA, actin, myosin, myelin basic protein (MBP) and tubulin.

7. Method according to either of Claims 5 and 6, **characterized in that** the clones identified in step b) are fused with a non-Ig-secreting myeloma.

8. Monoclonal antibody which can be obtained using the method according to one of Claims 5 to 7, **characterized in that** it is capable of inducing overexpression of IL-1Ra by macrophages or monocytes.

9. Clones of EBV B lymphocytes identified and amplified by the method according to one of Claims 5 to 7, **characterized in that** they produce monoclonal antibodies capable of inducing overexpression of IL-1Ra by macrophages or monocytes.

10. Monoclonal antibody obtained with a clone according to Claim 9, **characterized in that** it is capable of inducing overexpression of IL-1Ra by macrophages or monocytes.

11. Composition comprising an antibody according to one of Claims 1 to 4, 8 and 10 and a pharmaceutically acceptable vehicle..

12. Composition according to Claim 11, **characterized in that** it is suitable for intravenous, subcutaneous or intramuscular administration.

13. Method for preparing an IgG subpopulation, **characterized in that** it comprises the following steps:
a) preparing an insoluble support onto which DNP-lysine is grafted,
b) adsorbing polyvalent Igs onto the support obtained in step a),
c) eluting the Igs retained on the DNP portion bound to the support so as to collect the fraction which interacts with DNP,
d) selecting the fractions which exhibit reactivity with respect to given autoantigens, and
e) selecting the fractions which induce overexpression of IL-1Ra by macrophages or monocytes, at the secreted protein and mRNA level.

14. Method according to Claim 13, **characterized in that** step d) comprises measuring the reactivity for tetanus toxoid, taking the degree of enrichment relative to the activity of the initial polyvalent Igs as control value.

15. Method according to either of Claims 13 and 14, **characterized in that** step d) comprises an ELISA assay carried out on a panel of autoantigens selected in particular from DNA, actin, myosin, myelin basic protein (MBP) and tubulin.

16. Method according to one of Claims 13 to 15, **characterized in that** the Igs deposited on the solid support obtained in step a) are adsorbed either in the form of polyvalent IgGs which have been lyophilized and redissolved or which are in liquid form, or in the form of intermediate fractions obtained during a process for producing polyvalent IgGs in phosphate buffer comprising NaCl, the concentration of which can range from 0M to 3M.

17. Use of an IgG subpopulation which can be obtained using the method according to one of Claims 13 to 16, for producing a medicinal product for inducing overexpression of IL-1Ra in macrophages or monocytes.

18. Use according to Claim 17, **characterized in that** the IgG subpopulation is prepared from polyvalent Igs or any other intermediate fraction obtained during the method for producing IVIgs for therapeutic use.

19. Use of a monoclonal antibody according to one of Claims 1 to 4, 8 and 10, for preparing a medicinal product.

20. Use according to Claim 19, for preparing a medicinal product intended for treating and preventing inflammatory diseases.

21. Use according to Claim 19, for preparing a medicinal product intended for treating and preventing septicaemia and septic shock.

22. Use according to Claim 19, for preparing a medicinal product intended for treating and preventing arthritis, rheumatoid arthritis, polyarthritis, osteoporosis, inflammatory bowel diseases, autoimmune diseases, transplant rejection, ischaemia, traumas, in particular brain traumas, psoriasis, dermatoses, restenoses, respiratory diseases, allergic rhinitis, allergies and cancer.

23. Use according to Claim 17 or 18, for preparing a medicinal product intended for treating and preventing inflammatory diseases, septicaemia and septic shock, arthritis, rheumatoid arthritis, polyarthritis, osteoporosis, inflammatory bowel diseases, ischaemia, traumas, in particular brain traumas, psoriasis, dermatoses, restenoses, respiratory diseases, allergic rhinitis, allergies and cancer.
